(19) **Europäisches Patentamt**
**European Patent Office**
**Office européen des brevets**

(11) **EP 3 223 697 B1**

(12) **EUROPEAN PATENT SPECIFICATION**

(45) Date of publication and mention
of the grant of the patent:
**18.09.2024 Bulletin 2024/38**

(51) International Patent Classification (IPC):
**A61B 5/05** (2021.01)   **A61B 5/107** (2006.01)

(21) Application number: **15864096.1**

(22) Date of filing: **24.11.2015**

(52) Cooperative Patent Classification (CPC):
**A61B 5/0507; A61B 5/1075; A61B 5/442;**
**A61B 5/4519; A61B 5/4872; G01S 7/40;**
**G01S 13/86; G01S 13/867; G01S 13/88;**
**G01S 13/89**

(86) International application number:
**PCT/KR2015/012613**

(87) International publication number:
**WO 2016/085223 (02.06.2016 Gazette 2016/22)**

(54) **APPARATUS AND METHOD FOR ANALYZING BODY TISSUE LAYER IN ELECTRONIC DEVICE**

VORRICHTUNG UND VERFAHREN ZUR ANALYSE EINER KÖRPERGEWEBESCHICHT BEI EINER ELEKTRONISCHEN VORRICHTUNG

APPAREIL ET PROCÉDÉ POUR ANALYSER UNE COUCHE DE TISSU CORPOREL DANS UN DISPOSITIF ÉLECTRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.11.2014 RU 2014147150**
**28.10.2015 KR 20150150405**

(43) Date of publication of application:
**04.10.2017 Bulletin 2017/40**

(73) Proprietor: **Samsung Electronics Co., Ltd.**
**Suwon-si, Gyeonggi-do 16677 (KR)**

(72) Inventors:
• **CHERNOKALOV, Alexander Gennad'yevich**
**Korolev**
**Moscow reg. 141075 (RU)**
• **KLETSOV, Andrey Vladimirovich**
**125413 (RU)**

• **KHRIPKOV, Alexander Nikolayevich**
**Lobnya**
**Moscow region 141730 (RU)**
• **CHO, Jae-Geol**
**Yongin-si**
**Gyeonggi-do 16923 (KR)**

(74) Representative: **HGF**
**HGF Limited**
**1 City Walk**
**Leeds LS11 9DX (GB)**

(56) References cited:
**WO-A1-2014/045181    WO-A1-2015/114626**
**WO-A2-2005/044085    JP-A- 2013 158 634**
**KR-A- 20130 041 593    US-A- 6 061 589**
**US-A1- 2009 309 786    US-A1- 2009 322 349**
**US-A1- 2010 069 744    US-A1- 2011 230 738**
**US-A1- 2013 041 237    US-A1- 2014 114 196**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

The present disclosure relates to analysis of body tissue layers in an electronic device.

## Background Art

Personalized monitoring of health parameters has a vital priority for every human being: body fat mass monitoring, head imaging system for tumor detection, breast imaging system for breast cancer, heart functioning, and blood vessel movement analysis, among others, are of utmost importance for healthcare.

Central obesity is said to bring about lifestyle-related diseases, for example diabetes, hypertension, and hyperlipidemia. It could be effectively prevented by monitoring visceral fat, or fat that accumulates around the internal organs on the inner side of the abdominal muscles and the back muscles, and is distinct from the subcutaneous fat that is located toward the surface of the trunk area.

Until now, there are no appliances to periodically monitor fat thickness at home. Medical imaging utilizes 3D reconstruction systems, which require complex and expensive hardware and processing algorithm implementation. New methods are needed, that can detect changes of fat thickness with millimeter accuracy and that can be used for daily personal usage. A fat monitoring system is needed because extensive studies have demonstrated that early detection of obesity symptoms may lead to the most effective treatment.

In U.S. Patent No. 7,725,150 B2, a variant of UWB sensor known as a micropower impulse sensor combined with advanced signal processing techniques to provide a new type of medical imaging technology including frequency spectrum analysis and modern statistical filtering techniques to search for, acquire, track, or interrogate physiological data is described. Disadvantages of existing implementations, such as US 7,725,150 B2, may include the following:

The receiver is triggered by the delayed version of the base band pulse train; depth information analysis requires sequential sweep of the delay value within delay range. Data processing and statistical filtering is required for each delay value, thus the process is time consuming. The method for physiological data extraction requires a fixed position of the UWB sensor on the skin surface. Scanning of the physiological data along surface of the bodily organs is not supported.

This device is supposed to be fixedly placed above the area of interest and reconstruct the vital signals in time domain.

Displacement of the UWB sensor disrupts the measurement due to no synchronization provided between bodily organs depth scanning process (range finder mode) and mechanical displacement of the UWB sensor relatively to the surface. Therefore, scanning of the physiological data along a surface of the bodily organs is not supported. In this case a tissue structure image in 3D or 2D cannot be reconstructed.

It is impossible to measure physiological parameters during continuous movement of the UWB sensor along the human body surface.

A method for volume visualization in UWB sensor and a system thereof is described in U.S. Patent No. 8,089,396 B2. This patent describes method for measurement results processing and 3D data representation.

Following disadvantages of US 8,089,396 limit its applicability:

The stationary position of the UWB sensor relative to visualization volume limits the resolution of 3D visualization. Acceptable resolution is only achievable if antenna array structure has the same size as the entire volume to be visualized. Therefore fat scanning task will require bulky device size compared to size of entire human body.

Receiving antenna array of the disclosed UWB sensor cannot receive signals from an object located at its side due to shadowing effect. Therefore, usage of the UWB sensor of US 8,089,396 directly in touch with the human body is impossible.

With mentioned disadvantages, the method proposed in US 8,089,396 is not optimal for the applications disclosed in the present disclosure.

In patent document JP5224454, the plurality of transmit and receive antennas are fixed in predefined positions, surrounding fixed test volume. Body tissue must be tightly placed within that test volume. Human body phantom tissues are used for calibration of the measurement system of JP5224454. The test volume is completely filled with the human body phantom tissues during the calibration.

The following disadvantages of JP5224454 limit its applicability:

Antenna structure should have the same size as a body organ under imaging. Therefore fat scanning task will require a bulky device size compared to human body size.

Calibration with human body phantom tissues is required before the measurement that cannot be done at home conditions.

Fixed test volume should have specific size of corresponding human body part. Therefore, fat measurement at various body parts (i.e. belly, legs, hands, neck) is not possible.

Cancerous tissue detection is claimed; however normal tissue thickness measurement is a completely different task, which requires another measurement method.

In patent application document US 2010/0274145 A1 fetal and/or maternal monitoring devices, systems and methods using UWB medical sensor are described. A main application of this device is to detect vital signals. The following disadvantages limit its application for tissue structure visualization:

This device is supposed to be fixedly placed above the area of interest and re- construct the vital signals in time domain.

The receiver is triggered by the delayed version of the base band pulse train; depth information analysis requires sequential sweep of the delay value within delay range. Data processing and statistical filtering is required for each delay value, thus the process is time consuming. The method for physiological data extraction requires fixed position of the UWB sensor on the skin surface.

Displacement of the UWB sensor disrupts the measurement due to no synchronization provided between bodily organs depth scanning process (range finder mode) and mechanical displacement of the UWB sensor relatively to the surface. Therefore, scanning of the physiological data along surface of the bodily organs is not supported. In this case tissues structure image in 3D or 2D is cannot be reconstructed.

It is impossible to measure physiological parameters during continuous movement of the UWB sensor along the human body surface.

Several algorithms are available to reconstruct a 2D or 3D image from the collected data. A number of reconstruction algorithms are described in various literature, for example US. Pat. No. 6,061,589, Jack E. Bridges et al., Lopez-Sanchez, J. M., Fortuny-Guasch, 1., "3-D Radar Imaging using Range Migration Techniques," ISSN 0018-926X (IEEE Transactions on Antennas and Propagation, vol. 48, no. 5, May 2000). These algorithms are based on antenna characterization in far field zone using their radiation pattern and not applicable for analysis of near-field electromagnetic waves induced within the tissue layers.

US 2010/069744 discloses an imaging system for generating a three-dimensional image of a body part of a patient.

US 2009/309786 discloses a synthetic aperture radar system.

### Disclosure of Invention

### Solution to Problem

The invention is defined by the claims. According to an aspect of an exemplary example of the disclosure, a mobile device includes a receiver configured to receive signals radiated to an object and reflected from the object during a movement of the mobile device along a surface of the object; and at least one processor configured to identify positions of the mobile device during the movement of the device; and generate information on at least one tissue layer of the object based on the signals and relative positions of the mobile device, wherein the information is generated during the movement of the mobile device.

The object may include a body, wherein the at least one tissue layer may include at least one from among a muscle, a skin and a fat, and wherein the information may include a thickness associated with the at least one tissue layer.

The mobile device may further include: a transmitter configured to radiate the signals to the object while the electronic device moves along a surface of the object.

The mobile device may further include: a motion sensor configured to determine the plurality of positions while the electronic device moves.

The mobile device may further include: a display configured to display an image representing the information.

The mobile device may further include: a communicator configured to transmit the information to another electronic device.

The mobile device may further include: at least one antenna configured to radiate the signals and to detect the signals reflected from the object, and the at least one antenna may include flexible materials.

The mobile device may further include: a reference coupler configured to generate a marker signal for a calibration relating to a signal delay associated with the signals.

The controller may be further configured to measure a magnitude attenuation and a phase delay of the signals.

The information may be generated based on a magnitude attenuation and a phase delay of the signals, and an estimation of signal attenuation corresponding to a thickness of the at least one tissue layer.

According to another aspect of an exemplary example of the disclosure, a method for operating an mobile device includes receiving signals radiated to an object and reflected from the object during a movement of the mobile device along a surface of the object; identifying positions of the mobile device during movement of the mobile device; and generating information on at least one tissue layer of the object based on the signals and relative of the mobile device, wherein the information is generated during movement of the mobile device.

The object may include a body, and the at least one tissue layer may include at least one from among a muscle, a skin and a fat, and the information may include a thickness associated with the at least one tissue layer.

The method may further include: radiating the signals to the object while the electronic device moves along a surface of the object.

The method may further include: determining the plurality of positions while the electronic device moves.

The method may further include: displaying an image representing the information.

The method may further include transmitting the information to another electronic device.

The signals may be radiated and detected through at least one antenna, and the at least one antenna may include flexible materials.

The method may further include: generating a marker signal for a calibration relating to a signal delay associated with the signals.

The method may further include: measuring a magnitude attenuation and a phase delay of the signals.

The information may be generated based on a magnitude attenuation and a phase delay of the signals, and an estimation of signal attenuation corresponding to a thickness of the at least one tissue layer.

The present disclosure discloses microwave tissue layers profile determining and imaging device, which enables two dimensions (2D) or three dimensions (3D) "section" objects structure imaging for body tissue layers reconstruction. Also present disclosure discloses microwave imaging device, which displays the regions of visceral fat and subcutaneous fat and presents examination results in a visual form for easy understanding.

Ultra-wideband (UWB) healthcare or medical applications monitoring device is capable of non-invasive body tissue layers thickness profile measurement along the surface, the monitoring device includes a UWB microwave sensor comprising an microwave ultra-wideband transmit and receive antennas.

A related example to the disclosure relates to an ultra-wideband device for determining a profile of body tissue layers, the device comprising: an ultra-wideband sensor for obtaining tissue parameters information at a plurality of positions on the body, the ultra-wideband sensor is adapted for transmitting the microwave signals into the body using a transmit antenna of a ultra-wideband sensor and receiving reflected microwave signals from the body by a receive antenna of the ultra-wideband sensor; a motion sensor for detecting the plurality of positions during the movement of the ultra-wideband sensor along a surface of the body; and a controller for generating tissue parameters information along the surface of the body based on the ultra-wideband sensor signals at the plurality of positions during the movement of the ultra-wideband sensor and based on motion sensor signals at the plurality of positions and for determining the profile of body tissue layers based on the tissue parameters information.

Additional examples disclose that the motion sensor is capable to measure coordinates of the ultra-wideband sensor, obtained during movement of the ultra-wideband sensor along the surface of a body; the device is further configured for imaging the tissue parameters information or the profile of body tissue layers using a display; the ultra-wideband sensor further comprises transmitter block, receiver block; the transmitter block is intended for generation of continuous wave step-frequency or noise-like ultra-wide band spectrum signals conducted to the transmit antenna; the transmitter block is intended for generation of impulse or chirp pulse ultra-wide band spectrum signals conducted to the transmit antenna; the transmit antenna is intended for radiation of transmitted signals into the body; said transmit antenna is configured to minimize re-flections at the boundary antenna to the body skin; the receive antenna is intended for receiving reflected signals from the body; said transmit antenna is configured to minimize reflections at the boundary antenna to the body skin; ultra-wideband sensor is placed close to the body surface, but not necessary in direct contact with the skin;

transmit and receive antennas are adapted for defining spatial resolution by near-field focusing of transmitted and reflected signals; a reference coupler connected to the transmit antenna and to the receive antenna, and intended for transmitting of marker signals to the receive antenna; marker signals are intended for calibration of the microwave signals delays within the ultra-wideband sensor and identification of the skin surface as a "zero" depth level; the reference coupler is formed as a material with defined dielectric properties and thickness; said material is located between antennas and body surface; the receiver block is intended for detecting amplitude attenuation and phase delay of the received signals compared to the transmitted signal; the controller is intended for synchronization of the transmitter block, the receiver block and acquisition of amplitude attenuation and phase delay of the reflected signal data during movement of the mobile device along the body surface; the motion sensor is intended for transmitting position coordinates of the ultra-wideband sensor to the controller during movement of the ultra-wideband sensor along the body surface; the controller is intended for reconstruction of the living-body-tissue layers profile using attenuation and phase delay of the reflected signals and coordinates of the ultra-wideband sensor measured at a number of positions during its movement along the body surface; the controller is configured for performing reconstruction of the living-body-tissue layers profile using Fourier, inverse filtration, cepstral or related data processing methods; the controller is intended for reconstruction of the living-body-tissue layers profile, taking into account nonuniform and discontinuous movement of the ultra-wideband sensor; the controller is intended for real-time tuning of operating frequency range of the transmitter block and the receiver block, thus configuring maximum depth of the living-body-tissue layers profile determining; transmit and receive antennas functions are performed by a single antenna; transmit and receive antennas are placed together in a single assemble and cannot be moved one relatively to the other; transmit and receive antennas are fabricated using flexible materials such as a flexable printed circuit board (FPCB), an Indium tin oxide film or alike; said transmit and receive antennas could be flexibly moved one relatively to the other; the device is configured for conformal adaptation of its surface for the body during the manual movement of the ultra-wideband sensor along the body surface; the transmit antenna and the receive antenna are configured to move relatively to each other such that measurement accuracy for determining of living-body-tissue layers profile and layers thickness measurement is improved; the display is configured for indication of measurement results as a cross section of a living-body-tissues structure in 2D and/or 3D image style and/or thickness profile graph for living-body-tissues; the controller is intended for thickness measurement of a certain kind of living-body-tissues like fat tissue, or skin tissue, or muscle tissue or all of them; the device is embedded in consumer electronic device like smartphone, tablet computer, or any other wearable or mobile device; controller is embedded into as a part of the data processing block embedded into consumer electronic device; the device is implemented as an independent device.

Another related example to the disclosure relates to a method of non-contact determining a profile of body tissue layers, the method comprising generating microwave signals as a ultra-wide band spectrum signals using a controller; transmitting the microwave signals into the body using a transmit antenna of a ultra-wideband sensor; receiving reflected microwave signals from the body by a receive antenna of the ultra-wideband sensor; moving of the ultra-wide band sensor along a surface of a living body; determining a plurality of positions of the ultra-wideband sensor; determining amplitude and phase frequency characteristics of the reflected microwave signals at the plurality of positions using the controller when movement of the ultra-wide band sensor along a body surface; determining the profile of body tissue layers using information about the plurality of positions of the ultra-wideband sensor and information about the amplitude and phase frequency characteristics at the plurality of positions; wherein transmitting and receiving of microwave signals is performed at the plurality of positions during continuous movement of the ultra-wide band sensor on the body surface; and determining of the profile of the body tissue layers is performed by cumulative measurements from the plurality of positions during movement of the ultra-wide band sensor.

Additional example discloses that the method further includes imaging the determined profile of the body tissue layers using a display.

A technical result is simplified defining of the area of interest, simplifying body parameters determining in the selected area, increased speed of measurement of body parameters in the selected area, increased speed of the obtained data analysis.

Following data of body part under exploration is indicated: body fat percentage, body fat allocation, body fat volume within each body part separately. Fat volume allocation is indicated in 2D or 3D image.

Technical result of invention is achieved by using a ultra-wideband sensor which can be easy moved along a surface of a body in combination with a motion sensor for detecting position of the ultra-wideband sensor. Then data from the ultra-wideband sensor and the motion sensor are used for determining a profile of body tissue layers and imaging the tissue parameters.

**Brief Description of Drawings**

Fig. 1 illustrates a structure of an electronic device according to an exemplary embodiment.

Fig. 2A illustrates a structure of an electronic device implemented in a form of a combination of devices according to an exemplary embodiment.

Fig. 2B illustrates a structure of an electronic device implemented in an independent form according to an exemplary embodiment.

Fig. 3 illustrates operations of an electronic device according to an exemplary embodiment.

Fig. 4 illustrates a movement of an electronic device along a body surface according to an exemplary embodiment.

Fig. 5 illustrates a cross-section of body tissues and a movement of an electronic device during a measurement process according to an exemplary embodiment.

Fig. 6 illustrates a manual spiral or zigzag movement of an electronic device along a body surface, required for a three dimensional (3D) image reconstruction according to an exemplary embodiment.

Figs. 7A and 7B illustrate a radiation of a transmitted signal into a body, cross-section of the body is taken at center of a transmit antenna according to exemplary embodiments.

Fig. 8 illustrates a conformal adaptation of a sensor for a body shape according to an exemplary embodiment.

Fig. 9 illustrates a 3D simulation model for estimation of the maximum measurement depth of a sensor according to an exemplary embodiment.

Figs. 10A to 10C illustrate estimations of microwave signals attenuation for skin, fat and muscle tissues according to exemplary embodiments.

Fig. 11A illustrates a structure of an electronic device with a reference coupler for a calibration according to an exemplary embodiment.

Fig. 11B illustrates a structure of an electronic device with a calibration material for a calibration according to an exemplary embodiment.

Fig. 12 illustrates a measurement and data analysis procedure for body-tissue layer profile extraction.

Figs. 13A and 13B illustrate a body tissue layer structure that can be presented after a measurement according to exemplary embodiments.

Figs. 14A to 14D illustrate examples of 3D image reconstruction for a fat volume allocation according to exemplary embodiments.

**Best Mode for Carrying out the Invention**

The following description with reference to the accompanying drawings is provided to assist in a comprehensive understanding of exemplary embodiments of the disclosure and those exemplary embodiments defined by the claims and their equivalents. It includes various specific details to assist in that understanding but these are to be regarded as merely exemplary. Accordingly, those of ordinary skill in the art will recognize that various changes and modifications of the exemplary embodiments described herein can be made without departing from the scope and spirit of the disclosure. In addition, descriptions of well-known functions and constructions may be omitted for clarity and conciseness.

The terms and words used in the following description and claims are not limited to the bibliographical meanings, but are merely used to enable a clear and consistent understanding of the disclosure. Accordingly, it should be apparent to those skilled in the art that the following description of exemplary embodiments of the present disclosure is provided for illustration purpose only and not to limit the various exemplary embodiments of the disclosure, including those defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

By the term "substantially" it is meant that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide.

Exemplary embodiments of the present disclosure provide a technique for analyzing tissue layers of an object in an electronic device. Various exemplary embodiments relate to a field of microwave sensor, especially to non-contact UWB (ultra-wideband) body tissues sensor, in particular human body tissues sensor to be used for determining a profile of living body tissue layers and three dimensional (3D) or two dimensional (2D) medical imaging to visualize tissue structure under the skin surface and to define tissue layer thickness (e.g. fat, etc.).

Hereinafter, a term for indicating a signal, a term for indicating an object to be analyzed, and a term for indicating a component of the electronic device are illustrated to ease the understanding. Accordingly, the present disclosure is not limited to those terms mentioned, and can use other equivalent terms. For example, a body may be alternatively referred as a human body or a living body. However, various exemplary embodiments are not limited to a human or a living creature.

An exemplary embodiment provides a process for determining a profile of body tissue layers and tissue imaging and fat monitoring in consumer electronic devices like smartphones or tablet PC; thus, enabling healthcare and medical applications. Despite system simplicity, high image is provided resolution due to ultra-wide band (UWB) signal utilization and the necessity to visualize tissues on a small depth (around 5-10 cm). According to a radar theory, range (and image) measurement error is inversely proportional to signal bandwidth: $\delta T \sim \frac{1}{\sqrt{B}}$. Therefore, the UWB may provide a high image resolution. Further, the UWB signal is not harmful compared to a narrow band signal, because the signal energy is spread in a wider frequency band.

An exemplary embodiment may be realized by a consumer device with an integrated sensor, which allows measurement of tissue layer thickness by data processing from a series of positions during movement of the UWB sensor along the surface of the body.

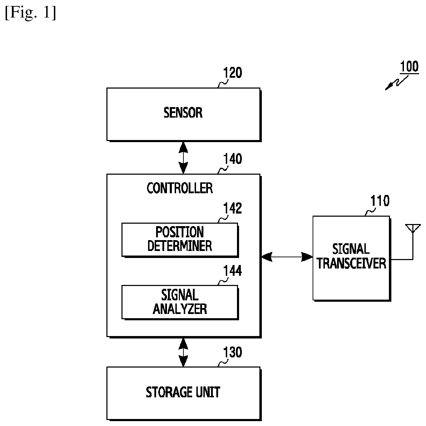

Fig. 1 illustrates a structure of an example electronic device according to an exemplary embodiment. Terms such as ~ unit' and '~ er/or' represent a unit for processing at least one function or operation, and can be implemented using hardware (e.g., a circuitry, a processor and so on), software, or a combination of hardware and software.

Referring Fig. 1, an electronic device 100 includes a signal transceiver 110, a sensor 120, a storage 130, and a controller 140.

The signal transceiver 110 transmits wireless signals through an least one antenna, and receives signals through the at least one antenna. The signal transceiver 110 may use a signal antenna to transmit and receive signals, or may use a transmit antenna and a receive antenna. The signal transceiver 110 may include a first module for transmissions and a second module for receptions. In an exemplary embodiment, the signal transceiver 110 radiates signals toward an object (e.g., a body) to analyze, and receives signals reflected from the object. Herein, the signals are configured by predefined values, and may be the UWB signals.

The sensor 120 measures data used to determine the position of the electronic device 100 during a movement of the electronic device 100. For example, the sensor 120 may include at least one sensing device such as an accelerometer, a camera or so on. The sensor 120 may be selectively activated according to a status of the electronic device 100. Conditions of an activation may be variously defined according to exemplary embodiments. In an exemplary embodiment, the sensor 120 may be activated when the signal transceiver 110 is operating. In another exemplary embodiment, the sensor 120 may be activated when the electronic device 100 is moving.

The storage 130 stores a basic program for operating the terminal, an application program, and data such as setting information. The storage 130 may be configured as a form of volatile memory, non-volatile memory or a combination thereof. Particularly, the storage 130 may store instructions for analyzing tissue layers of an object, data estimated by the sensor 120 and the signal transceiver 110, a result of the analysis and so on. The storage 130 provides the stored data according to a request of the controller 140.

The controller 140 controls overall operations of the electronic device. For example, the controller 140 transmits and receives the signals through signal transceiver 110. The controller 140 also controls estimation operations of the sensor 120. In addition, the controller 140 writes and reads data in the storage 130. The controller 140 may be implemented as at least one processor or at least one micro processor, or may be a part of any processor. Particularly, the controller 140 controls the electronic device to perform operations for analyzing the tissue layers according to various exemplary embodiments described hereafter. The controller 140 may include a position determiner 142 for determining positions of the electronic device and a signal analyzer 144 for analyzing reflected signals received by the signal transceiver 110.

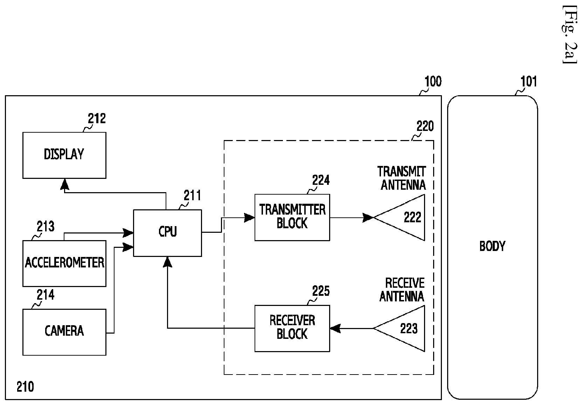

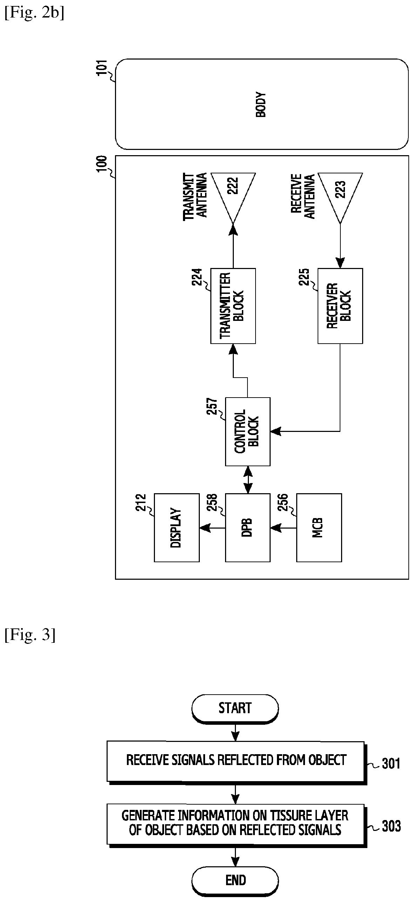

The electronic device 100 exemplified in Fig. 1 may analyze tissue layers of a body according to various exemplary embodiments. The electronic device 100 may be referred to as 'a sensor' or 'an UWB sensor'. The electronic device 100 may be implemented as a combination of a first device which needs an assistance from a second device (i.e., a smart phone, a tablet computer and so on) to analyze the tissue layers and the second device. The electronic device 100 may be implemented as a device which can operate independently. Fig 2A exemplifies an exemplary embodiment regarding the electronic device 100 implemented in a form of the combination, and Fig 2B exemplifies another exemplary embodiment regarding the electronic device 100 implemented in a standalone form.

Fig. 2A illustrates a structure of an electronic device implemented in a form of a combination of devices according to an exemplary embodiment. Fig. 2A illustrates a structure and functioning of a device 210 with an integrated UWB sensing module 220. That is, Fig. 2B illustrates the device 210 - a smartphone, a tablet computer, or any other wearable or mobile device, which includes the sensing module (sensor) 220. In an exemplary embodiment, the sensing module 220 is embedded into the device 210, and utilizes data processing and control modules included in the device 210.

Referring Fig. 2A, the electronic device 100 includes a device 210 and a sensing module 220. The device 210 includes a central processing unit (CPU) 211, a display 212, an accelerometer 212 and a camera 214. The sensing module 220 includes a transmit antenna 222, a receive antenna 223, a transmitter block 224 and a receiver block 225.

According to an exemplary embodiment, the following modules may be embedded into the device 210: an integrated circuit containing the transmitter block 224 and the receiver block 225; are the transmit antenna 222 and the receive antenna 223, connected with the transmitter block 224 and the receiver block 225. The transmit antenna 222 and the receive antennas 223 may be designed, for example, as slots and shapes in existing conductive parts of the device 210. The transmit antenna 222 may be directly connected to the output of the transmitter block 224 and the receive antenna 223 may be directly connected to the input of the receiver block 225. The transmitter block 224 generates microwave signals, which are conducted to the transmit antenna 222 and transmitted into the body 101. Signals reflected from the body 101 are received by the receive antenna 223 and detected by the receiver block 225. The receiver block 225 is intended for detecting amplitude attenuation and phase delay of the received signals compared to the transmitted signals.

The CPU 221 of the device 210 is used for the body tissues profile reconstruction. Operations of the transmitter block 224 and the receiver block 225 may be synchronized by the CPU 221. The CPU 221 may automatically preset the transmitter block 224 and the receiver block 225 for required measurement depth of body 101 tissues, power modes and other measurement parameters. The CPU 221 receives parameters of the reflected signal from the receiver block 225 and calculates structures of the body 101 tissues. Various implementations of the connections between CPU 221 and the transmitter block 224 and the receiver block 225 may be defined by the CPU 221 architecture, systems-on-chip implementation and peripheral interfaces.

The device 210 includes the accelerometer 213 and the camera 214, connected to the CPU 221 and intended for measurements of relative displacements. The accelerometer 213 and the camera 214 are used together for equidistant

7

depth measurements that allow the best result. In some exemplary embodiments, the accelerometer 213 or the camera 214 can be used separately or together for measurement of relative displacements. In these exemplary embodiments, the accelerometer 213 has the function of a motion control block that will be disclosed in more detail below. Image data from the camera 214 is transmitted to the CPU 221 of the device 210, information on relative position change is extracted using image processing algorithms. During measurement, the device 210 automatically detects its movement relatively to the body 101 surface by analyzing information from the accelerometer 213 and the camera 214. Position data is sent from the accelerometer 213 and the camera 214 to the CPU 221 to bind measurements with corresponding on-body positions of the device 210. The CPU 221 is intended for reconstruction of the living-body-tissue layers profile using attenuation and phase delay of the reflected signals and coordinates of the device 210 measured at a number of positions during movement of the device 210 along a surface of the body 101.

Measurement results are indicated on a display 212 of the device 210. Display 212 is connected to the CPU 221 and intended for representation of measurement results. As a result of data processing, CPU 221 is indicating on the display 212: the cross section (2D or 3D) of the body tissues thickness profile, information on the corresponding position on the body 101; fat layer thickness profile and other parameters regarding tissues of the body 101.

Fig. 2B illustrates a structure of an example electronic device implemented in an independent form according to an exemplary embodiment. Fig. 2B illustrates a structure and functioning of the UWB sensor as a standalone device, and a position of the UWB sensor above the skin surface.

Referring Fig. 2B, the electronic device 100 includes the transmit antenna 222, the receive antenna 223, the transmitter block 224, the receiver block 225, a motion control block (MCB) 256, a control block 257, a data processing block (DPB) 258, and a display 212.

The transmit antenna 222 and the receive antenna 223 are connected with the transmitter block 224 and the receiver block 225. Operations of the transmitter block 224 and the receiver block 225 may be synchronized by the control block 257. The control block 257 may automatically preset the transmitter block 224 and the receiver block 225 for required measurement depth of body 101 tissues, power modes and other measurement parameters. The control block 257 receives parameters of the reflected signal from the receiver block 225 and sends it to the DPB 258 to calculate structures of tissues of the body 101.

The electronic device 100 may be manually moved along the body 101 surface. During the measurement, the electronic device 100 automatically detects a movement of the electronic device 100 relatively to the body 101 surface using the MCB 256. The MCB 256 is capable of measuring coordinates of the ultra-wideband sensor, obtained during movement of the electronic device 100 along the surface of a body. MCB 256 is connected with DPB 258; MCB 256 sends data to DPB 258 to bind measurements with corresponding on-body positions of the electronic device 100.

The DPB 258 is intended for reconstruction of the living-body-tissue layers profile using attenuation and phase delay of the reflected signals and coordinates of the mobile device measured at a number of positions during movement of the electronic device 100 along a surface of the body 101. In addition, the DPB 258 may calculate fat layer thickness profile and other parameters of the body 101 tissues.

The display 212 may be connected to the DPB 258 and may be intended for representation of measurement results. As a result of data processing, the DPB 258 may send to the display 212 a cross section (2D or 3D) of the body tissues thickness profile including information on the corresponding position on the body 101.

In exemplary embodiments as shown in Figs 2A and 2B, the electronic device 100 may include a display (i.e., the display 212) to represent the result of an analysis on tissue layers. However, in another exemplary embodiment, the display is not included in the electronic device 100. In this case, to provide a user with the result of the analysis on the tissue layers, the electronic device 100 may transmit the result of the analysis or information regarding the result of the analysis to an external device capable of representing the result of the analysis. Accordingly, the electronic device 100 may include a communicator for transmitting signals to the external device. Herein, the information regarding the result of the analysis may be in the form of data or images.

According to various exemplary embodiments, the electronic device 100 analyzes the tissue layers while the electronic device 100 moves along a surface of the body 101. During the movement, signals are radiated from the transmit antenna 222 toward the body 101, and reflected signals from the body 101 are detected at the receive antenna 223. That is, components that may move along with the surface are the transmit antenna 222 and the receive antenna 223. Therefore, in some exemplary embodiments, in the structure of the electronic device 100, only some of the components including the transmit antenna 222 and the receive antenna 223 may be implemented in a movable form.

Fig. 3 illustrates operations of an electronic device according to an exemplary embodiment. Fig. 3 exemplifies a method for operating the electronic device 100.

Referring Fig. 3, at step 301, the electronic device 100 receives signals that are transmitted to an object and are reflected from the object. That is, the electronic device 100 transmits the measurement signals to the object, and receives reflected signals returned from the object. Receptions of the reflected signals are repetitively performed while the electronic device 100 moves. Herein, the measurement signals may for example be UWB signals. Further, a frequency band of the measurement signals may be in a industrial scientific and medical (ISM) band.

At step 303, the electronic device 100 generates information on tissue layers of the object based on the reflected signals. The information on the tissue layers may represent a thickness of tissues (i.e., a muscle, a skin and a fat). At this time, position information during a movement of the electronic device 100 may be used together to generate the information on the tissue layers. That is, the electronic device 100 generates information on tissue layers of the object based on the reflected signals and the position information estimated while the electronic device 100 moves.

According to various exemplary embodiments, non-contact measurements of various body parts may be performed. In an exemplary embodiment of the present disclosure, the electronic device 100 must be placed in front of the body 101. All body parts with any size and shape may be checked (i.e. belly, legs, hands, neck).

Living body tissues have a high contrast of dielectric permittivity values. For example, fat tissue permittivity may be ~4.7 and muscle tissue permittivity may be ~45. This almost 10 times difference may lead to high reflection coefficient from a border between tissues. Based on that physical phenomenon, the present disclosure discloses various exemplary embodiments for measuring borders between the fat layer and other layers (skin, muscle) of the body. As a result, good quality of living-body-tissue layers profile is obtained while keeping the emitted power of the electronic device 100 low, and maintaining a small size of the transmit antenna 222 and the receive antenna 223.

The measurement may done by a non-contact method. The transmit antenna 222 and the receive antenna 223 may be placed tight. However, it is not necessary to have electric contact to skin of the body 101 surface. Namely, a direct contact to the body 101 skin is not required. Any kind of light clothing, for example a t-shirt, may be placed between the body 101 surface and antennas 102 and 103 during measurement. In an exemplary embodiment of the present disclosure, the electronic device 100 may be manually moved along the body 101 surface.

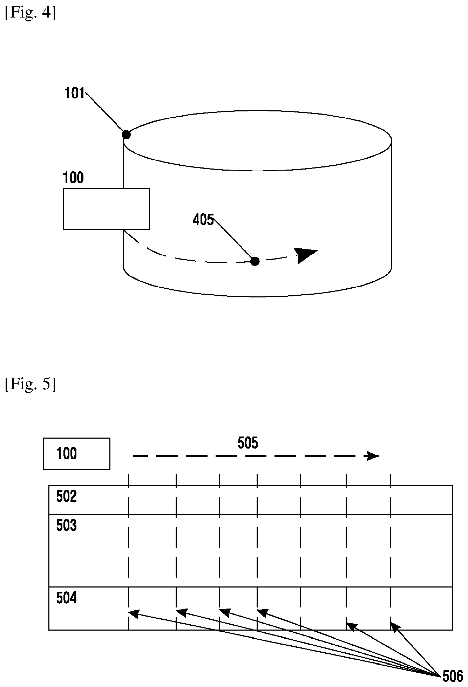

An example of a measurement process is described below. Fig. 4 illustrates an example of a movement of the electronic device 100 along a body surface. In an exemplary embodiment, as shown in Fig. 4, the electronic device 100 performs a series of measurements while moving along a path 405. Accordingly, the electronic device 100 is capable of forming a virtual antenna by moving. At that, structure of the body 101 tissues is calculated using measurement results taken at a number of positions with relative coordinates of these positions. This movement and measurement method achieves such accuracy, as if the electronic device 100 had a transmit 102 and receive 103 array antennas of large enough size to simultaneously cover all positions of the electronic device 100 moving along the path. That is, the electronic device 100 forms a virtual antenna using the movement. Therefore, various exemplary embodiments enable significant resolution improvement of the body tissues imaging without increasing a size of the electronic device 100.

An exemplary embodiment of the present disclosure utilizes the MCB 256 to locate a position at each measurement during scanning of the body tissue layers thickness profile. Measurement results from several different positions of the electronic device 100 are used for imaging of the body 101 tissues.

A measurement process is illustrated on Fig. 5 using a cross section of the body 101. Fig. 5 illustrates a cross-section of body tissues and a movement of an electronic device during a measurement process. Referring Fig. 5, the electronic device 100 moves along body skin surface 502. As an example, the body 101 includes a skin layer 502, a fat layer 503 and a muscle layer 504. The electronic device 100 is manually moved along the skin 502 surface in a direction 505 and makes a series of measurements at number of positions 506. For each of the measurements at each of the positons 506, the electronic device 100 may send a transmitted signal and receive a reflected signal. Movement of the electronic device 100 is continuously detected by the MCB 256, and position information is related to each measurement. After a movement 505 is complete, all measurements data are collected by the DPB 258. Image resolution improvement may be achieved by processed parameters of the received signal by the DPB 258 for multiple locations of the electronic device 100.

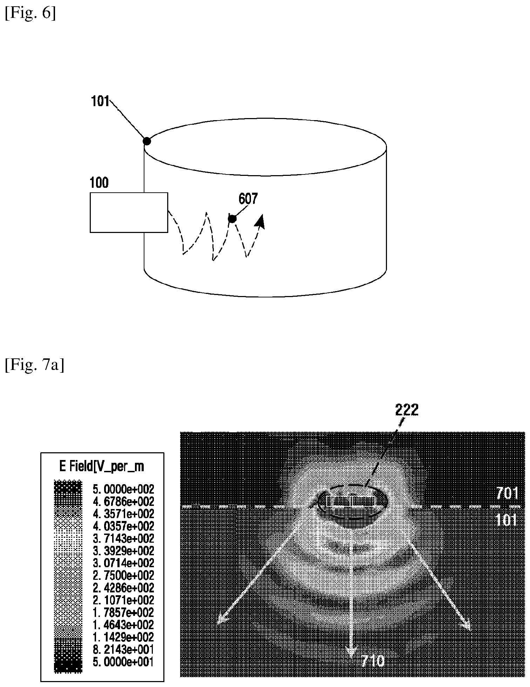

In the case that a 3D image must be reconstructed, the electronic device 100 may be moved on the body 101 surface in a spiral or zigzag path 607 depicted on Fig. 6. Fig. 6 illustrates an example of manual spiral or zigzag movement of an electronic device along a body surface, which may be required for a 3D image reconstruction. In this case, electronic device 100 covers area on the body 101 surface and gathers enough data to reconstruct a 3D image of the body tissues. Also, in this case, the MCB 256 tracks the movement along the surface and saves coordinates of multiple positions. Data processing for 2D and 3D reconstruction is described below.

A technical exemplary embodiment of the UWB sensor, that is, the electronic device 100 is described below.

In various exemplary embodiments of the present disclosure, the electronic device 100 can use different types of microwave signals as an ultra-wide band spectrum signals, for example:

- UWB impulse radio signal: impulse radio communicates with baseband pulses of very short duration, typically on the order of a nanosecond, thereby spreading the energy of the radio signal very thinly.
- chirp pulse UWB signal: a chirp may be a sinusoidal signal whose frequency increases or decreases over time.
- stepped frequency UWB signal: a variation of a chirp pulse when the signal frequency may be changed with several fixed frequency steps.
- noise-like UWB signal: UWB signal which may be generated by a deterministic system but have no periodic structure and look like white noise.

- maximum length binary sequence UWB signal: a type of pseudorandom binary sequence generated using maximal linear feedback shift registers, which may be an infinitely repeated sequence of a long random set of binary elements.

As it is clear to those skilled in the art, depending on microwave signals type to be used, appropriate signal transmitting and receiving technique must be realized. The transmitter block 224 and the receiver block 225 are configured to function using corresponding ultra-wide band spectrum signal. Resolution of body tissues imaging may be proportional to a bandwidth of a signal to be used. Hence, in an exemplary embodiment of the present disclosure, the UWB signals may be used.

For example, consider the usage of continuous wave stepped frequency modulation over a frequency band to make the UWB microwave spectrum. The received signal in time domain may be calculated from a frequency spectrum using an inverse Fourier transformation. While this method may offer enhanced resolution of body tissue imaging, the sensitivity may be limited by the fact that the electronic device 100 is continuously transmitting and receiving at the same frequencies. Parasitic coupling signals from the transmitter block 224 to the receiver block 225 may reduce the dynamic range of the receiver block 225. Thus maximum imaging depth of body 101 tissues is limited by decoupling of the transmit antenna 222 and the receive antenna 223.

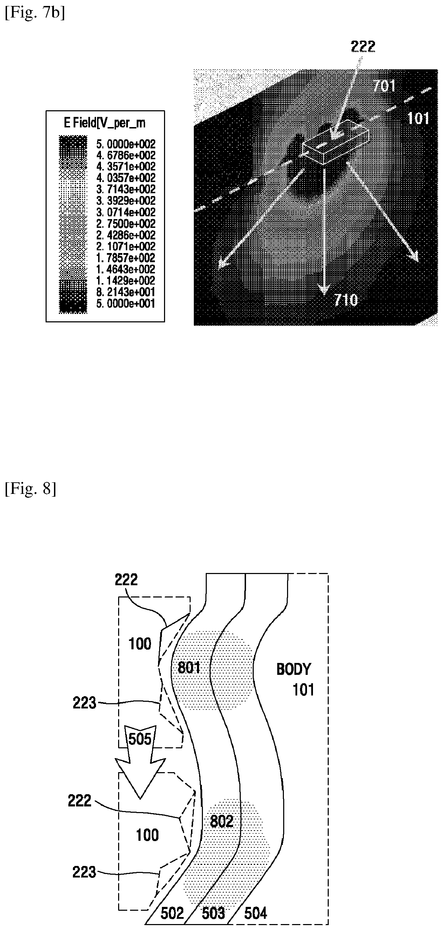

In an exemplary embodiment of the present disclosure, the transmit antenna 222 and the receive antenna 223 are configuring spatial resolution by near-field focusing of transmitted and reflected signals within an imaging area of body 101 tissues. Radiation of transmitted signal into the body 101 is illustrated in Figs. 7A and 7B. Figs. 7A and 7B illustrate examples of radiation of a transmitted signal into a body, where a cross-section of the body is taken at a center of a transmit antenna.

In Figs. 7A and 7B, a cross-section of the body 101 may be taken at a center of the transmit antenna 222. Intensity of an electric field in air 701 may be lower than an intensity of an electric field in the body 101; a radiation 710 of transmitted signal may be directed towards inner layers of body tissues. Therefore, parasitic back and side re-flections may be reduced.

In some exemplary embodiments of the present disclosure, the transmit antenna 222 and the receive antenna 223 are fabricated using flexible materials such as a flexible printed circuit board (FPCB), an Indium tin oxide film or alike. In that exemplary embodiment, the transmit antenna 222 and the receive antenna 223 could be flexibly moved one relatively to the other. Thus, a conformal adaptation for the body may be supported by the electronic device 100 as shown on Fig. 8.

Fig. 8 illustrates an example of a conformal adaptation of a sensor for a body shape. Referring Fig. 8, the transmit antenna 222 and the receive antenna 223 of the electronic device 100 flexibly move along with a surface of the body 101. Accordingly, the electronic device 100 can transmit and receive signals toward proper directions regarding the body 101. Therefore, an effective analysis of regions 801 and 802 may be received and archived or stored.

Antennas made of flexible material may bend around the body to provide stable gap thickness between antennas and skin (or, in some embodiments, cloth) surface during movement. In case of gap thickness stability parasitic reflections from body skin and cloth may also be stable and easy to remove.

In various exemplary embodiments, during the manual movement of the electronic device 100 along the body 101 surface, the transmit antenna 222 and the receive antenna 223 may conform to the body shape. This enables measurement of body tissue layers 502, 503 and 504 for every part of the body (i.e. belly, legs, hands, neck) regardless of its dimensions and curvature. Both flexible and rigid antennas can be used in through-cloth measurement, without electrical contact with skin. Also, conformal flexible antennas eliminate occurrence of air-filled gaps of variable thickness between antennas and the body, thus, minimizing reflections variation at the boundary antenna to the body skin (making it stable and simpler for removal). Cameras may be used for location determining similarly to common PC mouse tracking approach. Therefore, accuracy for image reconstruction of living-body-tissue layers profile and layers thickness measurement is improved by movement of the transmit antenna 222 relative to the receive antenna 223. This approach provides image reconstruction in the case that dielectric properties of tissue under investigation are undefined. Dielectric properties in this case can be defined by common data processing methods.

As is clear for those skilled in the art, high dielectric permittivity of fat and muscle tissues reduces wavelength in the body tissues by 3 - 7 times. Therefore, near-field focusing could be efficiently implemented using small-sized transmit antenna 222 and receive antenna 223.

In other exemplary embodiments of the present disclosure, the transmit antenna 222 and the receive antenna 223 may be placed together in a single assembly. Thus, maximum compactness of the electronic device 100 may be achievable. This implementation is intended for usage in tiny devices.

An accuracy may be estimated as described below.

An accuracy of the electronic device 100 may be defined as a depth (or vertical) accuracy and a horizontal accuracy. The depth accuracy may be defined as layer thickness variation which can be resolved. This accuracy may be proportional to wavelength at central frequency of the transmitted signal, generated by the transmitter block 224. The layer thickness variation can be confidently resolved if it is approximately $A_d = \lambda_0/3 \ldots \lambda_0/2$, where $\lambda_0$ is a wavelength in the body tissues 502 to 504. Here, $\lambda_0 \approx \lambda/Re(\sqrt{\varepsilon}')$, $\varepsilon'$ is dielectric permittivity. Variations of thickness smaller than Ad will not be resolved. For example, consider using UWB spectrum signals with center frequency f = 8 GHz, and measurement of the muscle layer with dielectric permittivity $\varepsilon'$= 40. Then a theoretical limit for depth accuracy may be Ad=0.0019m ($\lambda$=0.0375m, $\lambda$0=0.0059m).

Horizontal accuracy may depend on wavelength $\lambda$0, depth of the body tissue layers 502 - 504, and radiation pattern of the transmit antenna 222 and the receive antenna 223. Horizontal accuracy for the living-body-tissue layers profile extraction is proportional to Ah~ $\lambda$0. Hence in case if f=8 GHz, $\varepsilon'$=40 then Ah=0.0059m. That accuracy is sufficient to image the structure of sub-surface horizontal layers.

An example of a user scenario for measurement of body tissues with the UWB sensor, that is, the electronic device 100, is described below. According to an exemplary embodiment, a measurement procedure for subsurface body tissue layers thickness profile may be:

1) The user manually takes the device 100 and press an on-screen button "Start" button. After the user has pressed "Start" button, the device 100 may wait for the placement of device 100 on a body.
2) The user manually puts the electronic device 100 close to body surface under examination and moves the device 100 along the body keeping the close contact.
3) During close movement, the electronic device 100 is tracking its position and travelled distance using the MCB 258.
4) When the electronic device 100 identifies a "Finish" time, the device 100 processes the data using the CPU 211 or the DPB 258 to find a final result of the fat tissue thickness profile. After that, the electronic device 100 indicates obtained results on the display 212.
5) The user may move the electronic device 100 away from the body 101 and observe the fat thickness profile results on the display 212 of the electronic device 100. Results may be depicted in a form of graph of fat thickness profile related with on-body position, including total travelled distance.

The electronic device 100 distinguishes its placement on the body surface and distinguishes the moment or time when the user removes it away from body surface. The time of removal from the body surface may be identified as a measurement finish. For example, sensing of the placement is implemented via antennas impedance changes when antenna are placed on the body 101.

If user makes 2D imaging or 3D imaging, the user may move the electronic device 100 in a different path (straight as in 405 of Fig. 4 or zigzag as in 607 of Fig. 6). All of these paths may be distinguished by the MCB 258 of the electronic device 100 due to its possibility to detect on-body displacements in 2 axes.

An example of a maximum measurement depth of the electronic device 100 is described below.

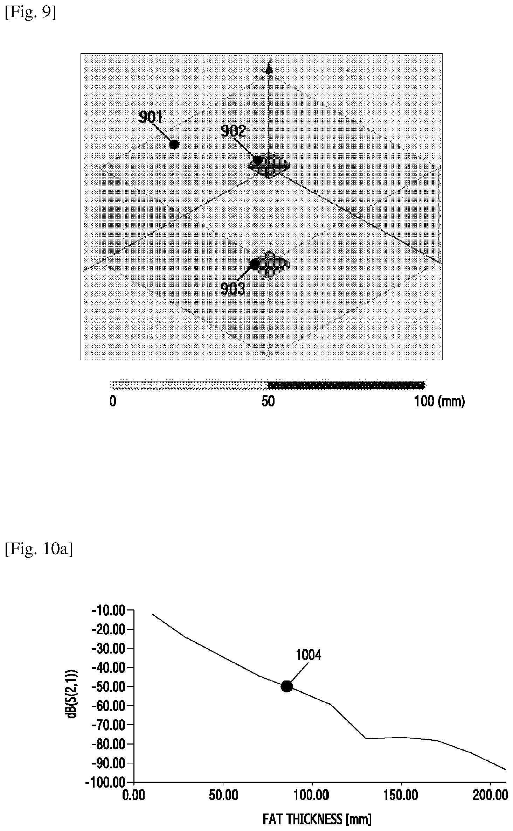

Fig. 9 illustrates an example of a 3D simulation model for estimation of a maximum measurement depth of a sensor. Fig. 9 exemplifies a 3D simulation model that was designed in order to estimate microwave signals attenuation in dependence of the body tissue types and thickness. Two antennas 902 and 903 were placed at opposite sides of a body phantom 901. The body phantom 901 thickness was variable. Antennas to be used in the 3D simulation model were bow-tie type with central feed point. Antennas size was 10×10×2.5 mm. Metal grounded shield at the antenna back side is placed to reduce backward radiation. Inside space of the antennas is filled with dielectric for impedance matching of antennas with the body tissues. Dielectric permittivity $\varepsilon$ = 4 was used for all simulations. Additionally, a thin 0.25mm polyester material was placed between antennas and tissue surfaces to simulate use-case of imaging through thin clothes.

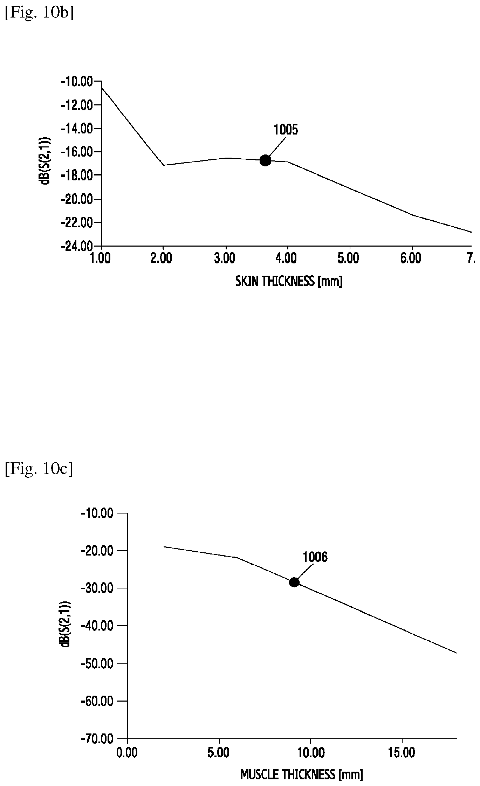

Figs. 10A to 10C illustrate examples of estimations of microwave signals attenuation for skin, fat and muscle tissues. Fig. 10A illustrates an example estimation of microwave signals attenuation for a skin 1005 at 8 GHz frequency, Fig. 10B illustrates an example estimation of microwave signals attenuation for a fat 1004 at 8 GHz frequency, and Fig. 10C illustrates an example estimation of microwave signals attenuation for a muscle 1006 at 8 GHz frequency. Referring Figs. 10A to 10C, the maximum depth of a body imaging by the electronic device 100 can be estimated based on characteristics of each tissue. For example, output peak power of the transmitter block 224 $P_{tx}$ = 0 dBm, transmit antenna 222 and the receive antenna 223 gain $G_{tx}=G_{rx}$=2 dBi, the receiver block 225 sensitivity $S_{rx}$=-60 dBm. In that case, the maximum attenuation $A_{ch}$ in tissue can be estimated as:

$$A_{ch} = P_{tx} + G_{tx} + G_{rx} - S_{rx} \qquad (1)$$

In Equation 1, $A_{Ch}$ denotes the maximum attenuation, Ptx denotes a transmit peak power, Gtx denotes a gain of the transmit antenna, and Grx denotes a gain of the transmit antenna.

In the considered example, $A_{ch}$ = 64 dB. Using results of Figs. 10A to 10C, the maximum scan depth at 8 GHz frequency can be estimated as $d_{skin}$> 7 mm, $d_{fat} \approx$ 57 mm, $d_{muscle} \approx$ 13 mm.

To improve an accuracy of analysis of the tissue layers, a calibration for the transmitter block 224 and the receiver block 225 of the electronic device 100 may be performed. The calibration for the layer tissues thickness measurement may be performed as described below.

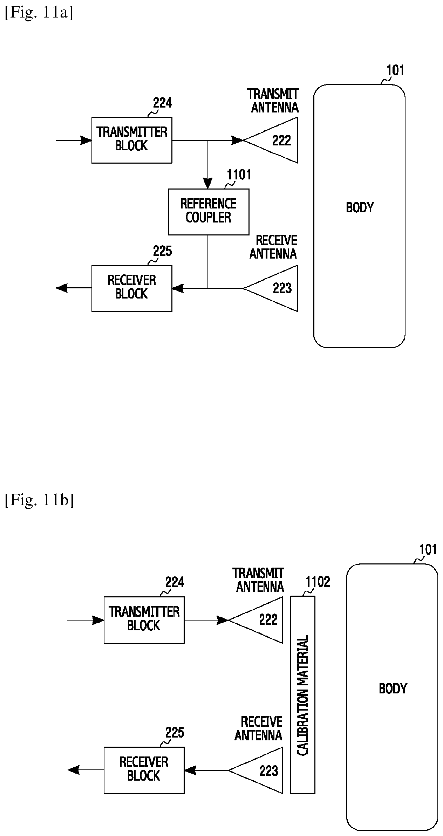

In some exemplary embodiments of the present disclosure, the electronic device 100 may include a reference coupler 1101 as shown in Fig. 11A. Fig. 11A illustrates an example structure of an electronic device with a reference coupler for a calibration. The reference coupler 1101 may be included for a calibration of a signal response from the skin surface, "zero" depth level. Input of the reference coupler 1101 is connected to the transmit antenna 222, output - to the receive antenna 223. The reference coupler 1101 is intended for forming the marker signals on output of the receive antenna 223 using attenuated transmitted signals. Said marker signals are added to the received signal and detected by the receiver block 225. Said marker signals are intended for calibration of the microwave signals delays within the electronic device 100.

In some exemplary embodiments of the present disclosure, a calibration of system response is performed using a calibration material 1102 placed within the gap between antennas 222 and 223 and the body 101, as shown in Fig. 11B. Fig. 11B illustrates a structure of an electronic device with a calibration material for a calibration. The calibration material 1102 may be included for a calibration of a signal response from the skin surface, "zero" depth level. The calibration material 1102 can be a plate of a homogeneous dielectric like FR-4. Signal reflections from the calibration material 1102 are predefined by known physical properties of the calibration material 1102.

In some exemplary embodiments of the present disclosure, marker signals are detected as a generally constant wave signal with minimum delay time. Actual signals received from the body are defined by subtracting detected marked signals from measured received signals.

Using the reference coupler 1101 or the calibration material 1102, boundary between transmit antenna 222 and the receive antenna 223 and the skin surface is identified as a "zero" depth level. The reference coupler 1101 or the calibration material 1102 allow to find a position of the reflected signal response from the skin surface. Thus, calibration procedure may be made automatically during the living-body-tissues reflection imaging. This calibration is also intended for parasitic reflection signals removal.

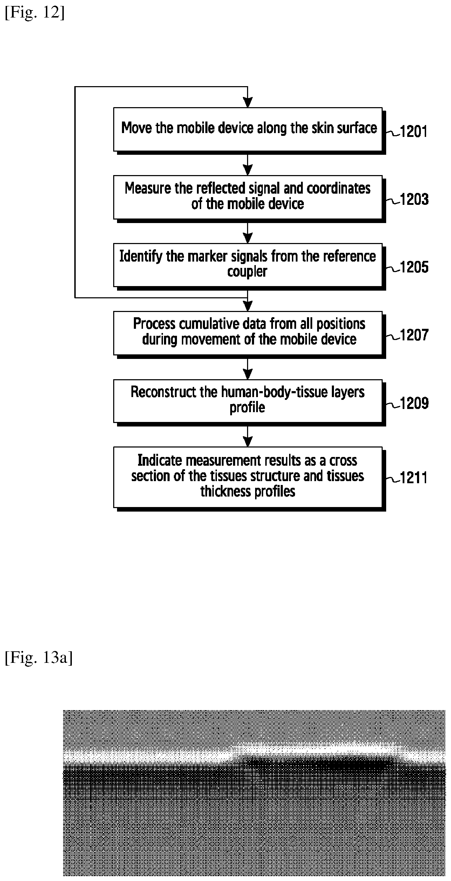

An example method of non-contact extraction of living-body-tissue layers profile using an ultra-wide band sensor for mobile health-care applications is illustrated in Fig. 12. Fig. 12 illustrates a measurement and a data analysis procedure for body-tissue layers profile extraction. An exemplary embodiment of the present disclosure may implement measurement and data analysis procedures as illustrated in Fig. 5.

Referring to the example method of Fig. 12, measurement is performed by placement of the electronic device 100 on a part of the body and manual movement of the electronic device 100 along the body surface (step 1201). During movement of the electronic device 100 along the body surface, measurement is performed at least at two positions as follows: the transmitter block 224 generates microwave signals as ultra-wide band spectrum signals; the transmitting antenna 222 radiates microwave signals into the body 101; the receive antenna 223 receives reflected signal from the body; the receiver block 225 detects amplitude and phase frequency characteristics of the reflected signal; the control block 257 receives data on amplitude attenuation and phase delay of the reflected signal from the receiver block 225.

The MCB 256 measures coordinates of positions of the electronic device 100 on the body 101 surface. Reflected signal parameters and coordinates of corresponding mobile device positions are sent to the DPB 258 (step 1203). The coordinates are measured in order to ensure that all measurements are made at equidistant intervals along the body. In a real device these coordinates can be for example a displacement in cm relative to a start position, or x and y displacement in cm on the body surface relative to a start point. The MCB 256 measures short time shifts (during ~ms time intervals) along the surface for example by integrating data from embedded 3-axis accelerometer (finding shift as square root from sum of squares of integrals of x, y, z data) or any other odometer sensor. After that the MCB summarizes all short time shifts to define said displacement from start position. The DPB 258, knowing real coordinates at which each measurement was made, may select equidistant measurements to provide correct image reconstruction. This technique may be used to perform successful image reconstruction even if a user moves the device non-uniformly or with variable speed along the body.

For each measurement, marker signals from the reference coupler 1101 are identified by the DPB 258 as reflected signal response from the skin surface, specifically, a "zero" depth level. This provides automatic real-time calibration during the living-body-tissues imaging (step 1205). After that, the electronic device 100 performs the step 1201 and step 1207.

The DPB 258 processes attenuation and phase delay of the reflected signals and coordinates of the mobile device measured at a number of positions during movement of the mobile device along the body surface. An image of the body tissue layers is formed by cumulative measurements from many positions. At that step, signal averaging is performed to take into account the mobile device movement non-uniformity and discontinuity (step 1207).

The data processing block performs image reconstruction of living-body-tissue layers profile and layers thickness measurement using aperture synthesis, Fourier, inverse filtration, cepstral or related data processing methods (step 1209).

12

At the final step of the measurement, the display 212 indicates the cross section (2D or 3D) of the body tissues thickness profile including information on the corresponding position on the body (step 1211).

An exemplary embodiment of a data processing technique for reconstruction of body tissues may performed as described below. The data processing by the UWB sensor, that is, the electronic device 100, may be split in several steps:

> 1. All datasets measured at specific on-body positions may be first converted to time domain. For example, if datasets was measured in frequency domain, first a Fourier transform may be applied to obtain time domain datasets.
> 2. Find and remove parasitic signals which are closest to zero depth level. These are the signals reflected not from the internal body tissues, but directly passed between transmitting and receiving antennas in the air, in skin, etc. After removal of parasitic signals, the datasets containing only pulses reflected from deep tissue borders may be obtained.
> 3. The datasets may be processed to find peak reflections data in each of datasets. Additional smoothing can be applied to peak reflections data.
> 4. Perform image reconstruction of living-body-tissue layers profile using aperture synthesis, Fourier, inverse filtration, cepstral or related data processing methods.
> 5. Perform layers thickness measurement by detecting depth of tissue boundaries (at least one) and show this to the user.

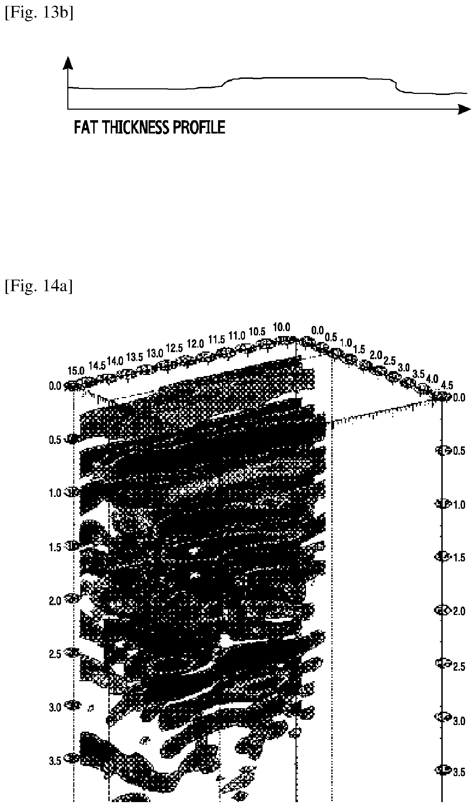

The electronic device 100 can depict a layered tissues structure in 2D after a user moves the electronic device 100 along with the skin surface. An example of a measurement result indicated by the electronic device 100 may be illustrated in Figs. 13A and 13B. Figs. 13A and 13B illustrate example body tissue layer structures that may be presented after a measurement. An exemplary embodiment can depict detailed structure of the body tissues in section-like view or like a profile graph of different tissue thickness.

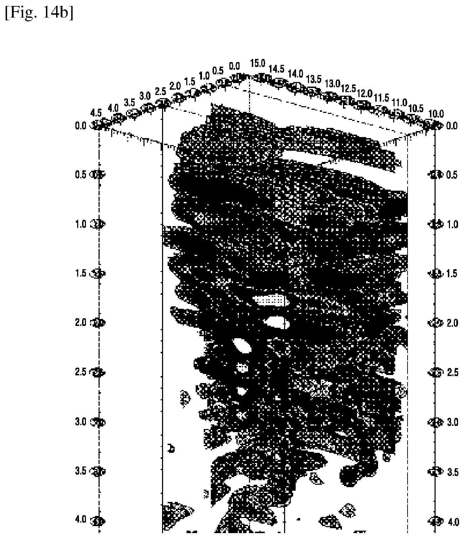

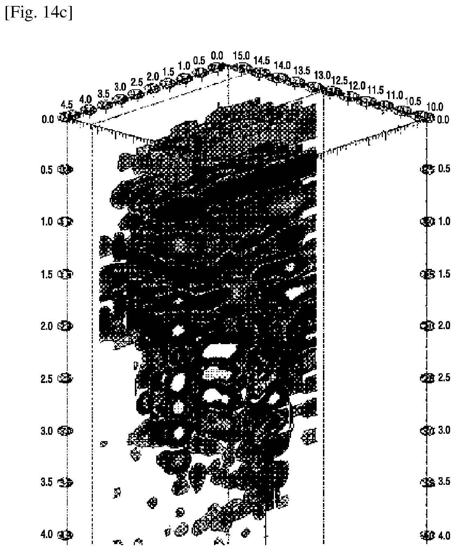

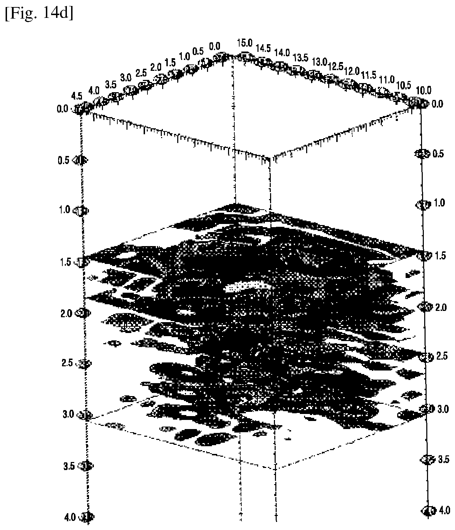

In an exemplary embodiment of the present disclosure, 3D reconstruction is implemented as a superposition of multiple 2D images taken for various cross-sections. 2D data processing may be applied in orthogonal dimensions, for example, in horizontal and vertical dimensions along the body. Data processing for 3D reconstruction requires a number of datasets measured at the body 101 surface with 10 mm average distance between measurement positions. Example of a 3D image reconstruction for the fat volume allocation is illustrated Figs. 14A-14D. Figs. 14A-14D illustrate examples of a 3D image reconstruction for a fat volume allocation.

In order to achieve the best accuracy, it may be important to provide measurements at known positions at the body skin surface. Information on body positions is also important for representation of finally processed datasets (peak reflection data) related with actual position of the sensor on the body skin surface.

Home-care and medical applications of analysis schemes for the tissue layers of the body are described. The analysis schemes may be applied for medical diagnosis applications by imaging of body organs inside the body 101. Dynamic tissue reconstruction of body organs and analysis of body organs functioning may be performed. In order to reconstruct the image, the electronic device 100 including the UWB sensor may make a series of measurements at number of positions along the body organ. Time duration of this measurement may be longer than average period of the organ movement.

Non-contact measuring technology for organ movements may have the following advantages: noninvasive method, infection-safe, and comfortable. It may be suitable for home-care continuous monitoring to indicate user's health and recovery status.

In some exemplary embodiments, the sensor identifies movement patterns of each part of the heart separately for cardiopulmonary sensing: heart strength, vascular age, arterial stiffness and other cardiovascular parameters.

In another exemplary embodiment, intestinal motility monitoring of contraction status is done for monitoring of intestine condition and disorders, such as recurrent obstruction, spasms and intestinal paralysis. Exemplary embodiments of the present disclosure provide non-invasive monitoring of physiological information, such as abdominal distension and recurrent obstruction. That enables home-care health monitoring and preliminary diagnosis.

Another useful feature of the UWB sensor is possibility of tissue differentiation. The UWB sensor can distinguish tissues on the basis of measured dielectric permittivity. The UWB sensor may detect tissue parameters if its antennas may be moved relatively to each other during measurement process. In some exemplary embodiments, a sensor may have a single transmit antenna and a series of electrically switchable receive antennas placed, for example, in a line. The UWB sensor may detect tissue permittivity from different signal propagation time between different pairs of transmitting and receiving antennas. Switchable approach provides single RF module use for multiple antennas and to simplify and reduce cost of a sensor. Also this approach may provide faster measurement and better accuracy due to avoiding the need for a user's manual sensor to move.

Example industrial applicability is described below. Aforementioned exemplary embodiments can find application in consumer electronic systems of the body tissues imaging sensors; in particular, it may provide the tissue thickness measurement and tissue 2D / 3D structure view to the depth of several centimeters. The claimed solution is especially suitable for use in the fields of healthcare and fitness consumer devices.

Example product applications as described below can be considered.

1. Precise tracking of body composition during a fitness course:

- Body fat allocation at body parts such as chest, abdominal area, thigh, lower back, bicep, neck, etc. Defining what person's body fat allocation means for his health status and what fitness strategy will give the best results.
- Tissue thickness profile, fat volume within each body part.
- Optimizing the fitness plan for the best way to improve person's body composition.
- Personalized goals definition and progress tracking.
- Obesity monitoring for prevention of lifestyle-related diseases: diabetes, hypertension, hyperlipidemia.

2. Reconstruction of body organs and their functioning, physiological parameters measurement:

- Head imaging system for tumors detection
- Breast imaging system for early detection of breast cancer
- Intestinal motility monitoring,
- Cardiopulmonary sensing: heart strength, vascular age, arterial stiffness
- Analysis of inner body organs: liver, kidney, etc.

An exemplary embodiment of the present disclosure may provide imaging capability by displaying the regions of visceral fat and subcutaneous fat. Examination results details may be shown visually for easy understanding. Subcutaneous fat may be measured directly by sensor and visceral fat can be estimated based on subtraction of subcutaneous fat amount from total body fat amount. Total body fat amount may be measured by common methods based on weight and height. In this case visceral fat measurement accuracy will be limited with common method accuracy.

The best imaging quality of living-body-tissue layers profile is obtained while keeping low emitted power of the UWB sensor and small-sized antennas. Achieved tissue thickness resolution accuracy is 2 mm.

Progress charts are stored and indicated for each body part within personalized health profile. This information is compared to reference data indicating overall health status of the person.

In an exemplary embodiment of the present disclosure, the display used for the indication is implemented as a screen of the mobile electronic device like a smartphone or tablet computer.

In some exemplary embodiments of the present disclosure, acquired health profile data is sent to personal doctor, physician or a coach.

Embodiments of the present invention according to the claims and description in the specification can be realized in the form of hardware, software or a combination of hardware and software.

Such software may be stored in a computer readable storage medium. The computer readable storage medium stores one or more programs (software modules), the one or more programs comprising instructions, which when executed by one or more processors in an electronic device, cause the electronic device to perform methods of the present invention.

Such software may be stored in the form of volatile or non-volatile storage such as, for example, a storage device like a Read Only Memory (ROM), or in the form of memory such as, for example, Random Access Memory (RAM), memory chips, device or integrated circuits or on an optically or magnetically readable medium such as, for example, a Compact Disc (CD), Digital Video Disc (DVD), magnetic disk or magnetic tape or the like. It will be appreciated that the storage devices and storage media are embodiments of machine-readable storage that are suitable for storing a program or programs comprising instructions that, when executed, implement embodiments of the present invention. Embodiments provide a program comprising code for implementing apparatus or a method as claimed in any one of the claims of this specification and a machine-readable storage storing such a program. Still further, such programs may be conveyed electronically via any medium such as a communication signal carried over a wired or wireless connection and embodiments suitably encompass the same.

While certain exemplary embodiments have been described, it will be understood by those skilled in the art that various changes in form and details may be made therein without departing from the scope as defined by the appended claims.

**Claims**

**1.**   A mobile device (100) comprising:

a sensor (120);
a display (212);
a receiver (225); and
at least one processor (140) configured to:

perform a measurement of tissue layers of an object (101) during a movement of the mobile device (100) by:

identifying (1203), by using the sensor (120), coordinates for each of a plurality of positions of the mobile device (100) during the movement of the mobile device (100);
measuring (301, 1203), by using the receiver (225), signals radiated to an object (101) and reflected from the object (101), at each of the plurality of positions of the mobile device (100); and
obtaining (303, 1207) thickness information for the tissue layers of the object (101) by processing, at each of the plurality of positions, an amplitude attenuation and a phase delay of the measured signals;

reconstruct (303, 1209) a structure image for the tissue layers of the object (101) based on the thickness information and the coordinates for each of the plurality of positions; and
control the display to display (1211) the structure image for the tissue layers of the object (101).

2. The mobile device of claim 1, wherein the thickness information for tissue layers are obtained based on a dielectric permittivity for each of a muscle, a skin and fat.

3. The mobile device of claim 1, further comprising:

a transmitter (224);
wherein the at least one processor is further configured to control the transmitter (224) to transmit the signals to the object (1010) during the movement of the mobile device (100).

4. The mobile device of claim 1, further comprising:

at least one antenna (222; 223) configured to radiate the signals and detect the signals reflected from the object (101),
wherein the at least one antenna (222; 223) is fabricated using flexible materials.

5. The mobile device of claim 1, wherein the at least one processor is, in order to obtain the structure image, configured to:

obtain multiple two-dimensional, 2D, images on a plurality of cross-sections based on the thickness information;
perform a three-dimensional, 3D, image reconstruction based on a superposition of the multiple 2D images; and
obtain the structure image based on the 3D image reconstruction.

6. The mobile device of claim 1, further comprising:

a reference coupler (1101) configured to generate a marker signal for a calibration regarding a signal delay associated with the measured signals,
wherein the at least one processor is further configured to perform the calibration based on a removal of the marker signal from the measured signals,
wherein the marker signal corresponds to a zero as a depth level.

7. The mobile device of claim 1,
wherein the coordinates are identified as equidistant intervals for the object (101).

8. A method for operating a mobile device (100), the method comprising:

performing a measurement of tissue layers of an object (101) during a movement of the mobile device (100) by:

identifying coordinates for each of a plurality of positions of the mobile device (100) during the movement of the mobile device (100); and
measuring signals radiated to the object (101) and reflected from the object (101) at each of the plurality of positions of the mobile device (100);

obtaining thickness information for the tissue layers of the object (101) by processing, at each of the plurality of positions, an amplitude attenuation and a phase delay of the measured signals;

reconstructing a structure image for the tissue layers of the object (101) based on the thickness information and the coordinates for each of the plurality of positions; and
displaying the structure image for the tissue layers of the object (101).

9. The method of claim 8,
wherein the thickness information for tissue layers is obtained based on a dielectric permittivity for each of a muscle, a skin and fat.

10. The method of claim 8, further comprising:

transmitting the signals during the movement of the mobile device (100); and
transmitting the thickness information to another electronic device.

11. The method of claim 8, wherein the signals are radiated and detected through at least one antenna (222; 223), and wherein the at least one antenna (222; 223) is fabricated using flexible materials.

12. The method of claim 8, wherein obtaining the structure image comprises:

obtaining multiple two-dimensional, 2D, images on a plurality of cross-sections based on the thickness information and the coordinates for each of the plurality of positions;
performing a three-dimensional, 3D, image reconstruction based on a superposition of the multiple 2D images; and
obtaining the structure image based on the 3D image reconstruction.

13. The method of claim 8, further comprising:

generating a marker signal for a calibration regarding a signal delay associated with the signals; and
performing the calibration based on a removal of the marker signal from the measured signals,
wherein the marker signal corresponds to a zero as a depth level.

14. The method of claim 8,
wherein the coordinates are identified as equidistant intervals for the object (101).


**Patentansprüche**

1. Mobile Vorrichtung (100), umfassend:

einen Sensor (120);
eine Anzeige (212);
einen Empfänger (225); und
mindestens einen Prozessor (140), der konfiguriert ist zum:
Durchführen einer Messung von Gewebeschichten eines Objekts (101) während einer Bewegung der mobilen Vorrichtung (100) durch:

Identifizieren (1203), durch Verwendung des Sensors (120), von Koordinaten für jede einer Vielzahl von Positionen der mobilen Vorrichtung (100) während der Bewegung der mobilen Vorrichtung (100);
Messen (301, 1203), durch Verwendung des Empfängers (225), von Signalen, die an ein Objekt (101) ausgestrahlt und von dem Objekt (101) reflektiert werden, an jeder der Vielzahl von Positionen der mobilen Vorrichtung (100); und
Erhalten (303, 1207) von Dickeninformationen für die Gewebeschichten des Objekts (101) durch Verarbeiten, an jeder der Vielzahl von Positionen, einer Amplitudendämpfung und einer Phasenverzögerung der gemessenen Signale;
Rekonstruieren (303, 1209) eines Strukturbilds für die Gewebeschichten des Objekts (101) basierend auf den Dickeninformationen und den Koordinaten für jede der Vielzahl von Positionen; und

Steuern der Anzeige zum Anzeigen (1211) des Strukturbilds für die Gewebeschichten des Objekts (101).

2. Mobile Vorrichtung nach Anspruch 1, wobei die Dickeninformationen für Gewebeschichten basierend auf einer dielektrischen Permittivität für jeweils einen Muskel, eine Haut und Fett erhalten werden.

3. Mobile Vorrichtung nach Anspruch 1, ferner umfassend:

   einen Sender (224);
   wobei der mindestens eine Prozessor ferner dazu konfiguriert ist, den Sender (224) zu steuern, um die Signale während der Bewegung der mobilen Vorrichtung (100) an das Objekt (1010) zu übertragen.

4. Mobile Vorrichtung nach Anspruch 1, ferner umfassend:

   mindestens eine Antenne (222; 223), die dazu konfiguriert ist, die Signale auszustrahlen und die von dem Objekt (101) reflektierten Signale zu detektieren,
   wobei die mindestens eine Antenne (222; 223) unter Verwendung flexibler Materialien hergestellt ist.

5. Mobile Vorrichtung nach Anspruch 1, wobei der mindestens eine Prozessor, zum Erhalten des Strukturbilds, konfiguriert ist zum:

   Erhalten multipler zweidimensionaler, 2D-, Bilder, an einer Vielzahl von Querschnitten basierend auf den Dickeninformationen;
   Durchführen einer dreidimensionalen, 3D-, Bildrekonstruktion basierend auf einer Überlagerung der multiplen 2D-Bilder; und
   Erhalten des Strukturbilds basierend auf der 3D-Bildrekonstruktion.

6. Mobile Vorrichtung nach Anspruch 1, ferner umfassend:

   einen Referenzkoppler (1101), der konfiguriert ist zum Erzeugen eines Markierungssignals für eine Kalibrierung bezüglich einer mit den gemessenen Signalen assoziierten Signalverzögerung,
   wobei der mindestens eine Prozessor ferner dazu konfiguriert ist, die Kalibrierung basierend auf einem Entfernen des Markierungssignals aus den gemessenen Signalen durchzuführen,
   wobei das Markierungssignal einer Null als Tiefenniveau entspricht.

7. Mobile Vorrichtung nach Anspruch 1,
   wobei die Koordinaten als äquidistante Intervalle für das Objekt (101) identifiziert werden.

8. Verfahren zum Betreiben einer mobilen Vorrichtung (100), wobei das Verfahren Folgendes umfasst:
   Durchführen einer Messung von Gewebeschichten eines Objekts (101) während einer Bewegung der mobilen Vorrichtung (100) durch:

   Identifizieren von Koordinaten für jede einer Vielzahl von Positionen der mobilen Vorrichtung (100) während der Bewegung der mobilen Vorrichtung (100); und
   Messen von Signalen, die an das Objekt (101) ausgestrahlt und von dem Objekt (101) reflektiert werden, an jeder der Vielzahl von Positionen der mobilen Vorrichtung (100);
   Erhalten von Dickeninformationen für die Gewebeschichten des Objekts (101) durch Verarbeiten, an jeder der Vielzahl von Positionen, einer Amplitudendämpfung und einer Phasenverzögerung der gemessenen Signale;
   Rekonstruieren eines Strukturbilds für die Gewebeschichten des Objekts (101) basierend auf den Dickeninformationen und den Koordinaten für jede der Vielzahl von Positionen; und
   Anzeigen des Strukturbilds für die Gewebeschichten des Objekts (101).

9. Verfahren nach Anspruch 8,
   wobei die Dickeninformationen für Gewebeschichten basierend auf einer dielektrischen Permittivität für jeweils einen Muskel, eine Haut und Fett erhalten werden.

10. Verfahren nach Anspruch 8, ferner umfassend:
    Übertragen der Signale während der Bewegung der mobilen Vorrichtung (100); und Übertragen der Dickeninformationen an eine andere elektronische Vorrichtung.

**11.** Verfahren nach Anspruch 8, wobei die Signale durch mindestens eine Antenne (222; 223) ausgestrahlt und detektiert werden, und
wobei die mindestens eine Antenne (222; 223) unter Verwendung flexibler Materialien hergestellt ist.

**12.** Verfahren nach Anspruch 8, wobei das Erhalten des Strukturbilds Folgendes umfasst:

Erhalten multipler zweidimensionaler, 2D-, Bilder an einer Vielzahl von Querschnitten basierend auf den Dickeninformationen und den Koordinaten für jede der Vielzahl von Positionen;
Durchführen einer dreidimensionalen, 3D-, Bildrekonstruktion basierend auf einer Überlagerung der multiplen 2D-Bilder; und
Erhalten des Strukturbilds basierend auf der 3D-Bildrekonstruktion.

**13.** Verfahren nach Anspruch 8, ferner umfassend:

Erzeugen eines Markierungssignals für eine Kalibrierung bezüglich einer mit den Signalen assoziierten Signalverzögerung; und
Durchführen der Kalibrierung basierend auf einem Entfernen des Markierungssignals aus den gemessenen Signalen,
wobei das Markierungssignal einer Null als Tiefenniveau entspricht.

**14.** Verfahren nach Anspruch 8,
wobei die Koordinaten als äquidistante Intervalle für das Objekt (101) identifiziert werden.

**Revendications**

**1.** Dispositif mobile (100) comprenant :

un capteur (120) ;
un dispositif d'affichage (212) ;
un récepteur (225) ; et
au moins un processeur (140) configuré pour :

réaliser une mesure des couches de tissu d'un objet (101) au cours d'un déplacement du dispositif mobile (100) par :

identification (1203), à l'aide du capteur (120), des coordonnées pour chacune d'une pluralité de positions du dispositif mobile (100) au cours du déplacement du dispositif mobile (100) ;
mesure (301, 1203), à l'aide du récepteur (225), des signaux émis vers un objet (101) et réfléchis par l'objet (101), à chacune de la pluralité de positions du dispositif mobile (100) ; et
obtention (303, 1207) d'informations d'épaisseur pour les couches de tissus de l'objet (101) en traitant, au niveau de chacune de la pluralité de positions, l'atténuation d'amplitude et le retard de phase des signaux mesurés ;

reconstruire (303, 1209) une image de structure pour les couches de tissu de l'objet (101) sur la base des informations d'épaisseur et des coordonnées pour chacune de la pluralité de positions ; et
commander au dispositif d'affichage d'afficher (1211) l'image de structure pour les couches de tissu de l'objet (101).

**2.** Dispositif mobile selon la revendication 1, lesdites informations d'épaisseur pour les couches de tissu étant obtenues sur la base d'une permittivité diélectrique pour chaque élément parmi un muscle, une peau et une graisse.

**3.** Dispositif mobile selon la revendication 1, comprenant en outre :

un émetteur (224) ;
ledit au moins un processeur étant en outre configuré pour commander à l'émetteur (224) de transmettre les signaux à l'objet (1010) au cours du déplacement du dispositif mobile (100).

**4.** Dispositif mobile selon la revendication 1, comprenant en outre :

au moins une antenne (222 ; 223) configurée pour émettre les signaux et détecter les signaux réfléchis par l'objet (101),
ladite au moins une antenne (222 ; 223) étant fabriquée à l'aide de matériaux souples.

**5.** Dispositif mobile selon la revendication 1, ledit au moins un processeur étant, afin d'obtenir l'image de structure, configuré pour :

obtenir de multiples images bidimensionnelles, 2D, sur une pluralité de sections transversales sur la base des informations d'épaisseur ;
réaliser une reconstruction d'image tridimensionnelle, 3D, sur la base d'une superposition des multiples images 2D ; et
obtenir l'image de structure sur la base de la reconstruction d'image 3D.

**6.** Dispositif mobile selon la revendication 1, comprenant en outre :

un coupleur de référence (1101) configuré pour générer un signal marqueur pour un étalonnage concernant un retard de signal associé aux signaux mesurés,
ledit au moins un processeur étant en outre configuré pour réaliser l'étalonnage sur la base d'une suppression du signal marqueur des signaux mesurés,
ledit signal marqueur correspondant au niveau zéro en tant que niveau de profondeur.

**7.** Dispositif mobile selon la revendication 1,
lesdites coordonnées étant identifiées sous forme d'intervalles équidistants pour l'objet (101).

**8.** Procédé permettant l'exploitation d'un dispositif mobile (100), le procédé comprenant :

la réalisation d'une mesure des couches de tissu d'un objet (101) au cours d'un déplacement du dispositif mobile (100) par :

identification des coordonnées pour chacune d'une pluralité de positions du dispositif mobile (100) au cours du déplacement du dispositif mobile (100) ; et
mesure des signaux émis vers l'objet (101) et réfléchis par l'objet (101) à chacune de la pluralité de positions du dispositif mobile (100) ;
obtention des informations d'épaisseur pour les couches de tissu de l'objet (101) en traitant, au niveau de chacune de la pluralité de positions, l'atténuation d'amplitude et le retard de phase des signaux mesurés ;

la reconstruction d'une image de structure pour les couches de tissu de l'objet (101) sur la base des informations d'épaisseur et des coordonnées pour chacune de la pluralité de positions ; et
l'affichage de l'image de structure pour les couches de tissu de l'objet (101).

**9.** Procédé selon la revendication 8,
lesdites informations d'épaisseur pour les couches de tissu étant obtenues sur la base d'une permittivité diélectrique pour chaque élément parmi un muscle, une peau et une graisse.

**10.** Procédé selon la revendication 8, comprenant en outre :

la transmission des signaux au cours du déplacement du dispositif mobile (100) ; et
la transmission des informations d'épaisseur à un autre dispositif électronique.

**11.** Procédé selon la revendication 8, lesdits signaux étant émis et détectés par l'intermédiaire d'au moins une antenne (222 ; 223), et
ladite au moins une antenne (222 ; 223) étant fabriquée à l'aide de matériaux souples.

**12.** Procédé selon la revendication 8, ladite obtention de l'image de structure comprenant :

l'obtention de multiples images bidimensionnelles, 2D, sur une pluralité de sections transversales sur la base des informations d'épaisseur et des coordonnées pour chacune de la pluralité de positions ;
la réalisation d'une reconstruction d'image tridimensionnelle, 3D, sur la base d'une superposition des multiples images 2D ; et
l'obtention de l'image de structure sur la base de la reconstruction d'image 3D.

13. Procédé selon la revendication 8, comprenant en outre :

la génération d'un signal marqueur pour un étalonnage concernant le retard de signal associé aux signaux ; et
la réalisation de l'étalonnage sur la base de la suppression du signal marqueur des signaux mesurés,
ledit signal marqueur correspondant au niveau zéro en tant que niveau de profondeur.

14. Procédé selon la revendication 8,
lesdites coordonnées étant identifiées sous forme d'intervalles équidistants pour l'objet (101).

[Fig. 1]

[Fig. 2a]

[Fig. 2b]

[Fig. 3]

START

RECEIVE SIGNALS REFLECTED FROM OBJECT ～301

GENERATE INFORMATION ON TISSURE LAYER OF OBJECT BASED ON REFLECTED SIGNALS ～303

END

[Fig. 4]

[Fig. 5]

[Fig. 6]

[Fig. 7a]

[Fig. 7b]

[Fig. 8]

[Fig. 9]

[Fig. 10a]

[Fig. 10b]

[Fig. 10c]

[Fig. 11a]

[Fig. 11b]

[Fig. 12]

- Move the mobile device along the skin surface — 1201
- Measure the reflected signal and coordinates of the mobile device — 1203
- Identify the marker signals from the reference coupler — 1205
- Process cumulative data from all positions during movement of the mobile device — 1207
- Reconstruct the human-body-tissue layers profile — 1209
- Indicate measurement results as a cross section of the tissues structure and tissues thickness profiles — 1211

[Fig. 13a]

[Fig. 13b]

FAT THICKNESS PROFILE

[Fig. 14a]

[Fig. 14b]

[Fig. 14c]

[Fig. 14d]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7725150 B2
- US 8089396 B2
- US 8089396 B
- JP 5224454 B

- US 20100274145 A1
- US 6061589 A
- US 2010069744 A
- US 2009309786 A

**Non-patent literature cited in the description**

- **JACK E. BRIDGES ; LOPEZ-SANCHEZ, J. M ; FORTUNY-GUASCH, 1. et al.** 3-D Radar Imaging using Range Migration Techniques. *IEEE Transactions on Antennas and Propagation,* May 2000, vol. 48 (5), ISSN 0018-926X